# EUROPEAN PATENT APPLICATION

(11) **EP 0 950 660 A1**
(43) Date of publication of application: **20.10.1999**
(21) Application number: 98200897.1
(22) Date of filing: 20.03.1998
(51) Int. Cl.: C07D 499/42

(54) **A process for recovery of 6-aminopenicillanic acid from a mother liquor**

(71) Applicant: GIST-BROCADES B.V., 2600 MA Delft (NL)
(72) Inventor: van der Does, Thomas, 2613 ZA Delft (NL); Kuipers, Rienk Hendrik, 2804 KZ Gouda (NL); Kerkhof, Johannes H.P.M., 3181 JD Rozenburg (NL); Kwant, Gerard Jan, 2631 JJ Nootdorp (NL)
(74) Representative: Visser-Luirink, Gesina, Dr.

(57) **Abstract**

The invention relates to a process for recovering 6-aminopenicillanic acid from a mother liquor, wherein 6-aminopenicillanic acid is extracted from the mother liquor with a recovery solution, which recovery solution is collected and re-used after 6-aminopenicillanic acid is isolated therefrom.

## Description

The invention is concerned with a process for recovery of 6-aminopenicillanic acid from a mother liquor.

6-Aminopenicillanic acid (6-APA) is a β-lactam nucleus, which is an important intermediate in the production of commercial β-lactam antibiotics, such as ampicillin, amoxicillin, epicillin, and ticarcillin. It has been proven feasible to prepare β-lactam antibiotics in so called enzymatic semi-synthetic routes. These routes involve enzymatically catalyzed processes and lack many of the disadvantages of the conventional synthetic methods for preparing β-lactam antibiotics. The enzymatic catalyzed reactions are highly selective, thus the production of many by-products, and the effluent and purification problems, which result therefrom, are avoided. Furthermore, enzymatic processes can be performed in aqueous environment.

The semi-synthetic routes to β-lactam antibiotics mostly start from fermentation products, such as penicillin G (Pen G). In a first step, penicillin G may be enzymatically converted to 6-APA, for instance in a manner as has been disclosed in Matsumoto (Bioprocess. Technol., 16, (1993), 67-88), Shewale and Sivaraman (Process Biochemistry, August (1989), 146-154), Savidge (Biotechnology of Industrial Antibiotics (Ed. E.J. Vandamme) Marcel Dekker, New York, (1984)), or Shewale et al. (Process Biochemistry International, June (1990), 97-103). The obtained 6-APA may subsequently be converted to a desired antibiotic by coupling to a suitable side chain, as has been described in *inter alia* EP-A-0 339 751, JP-A-53 005 185 and CH-A-640 240.

In the above described first step, wherein 6-APA is prepared, the product is eventually obtained in an aqueous solution from which it is crystallized. A serious problem of this crystallization is that up to 10% of the theoretical yield of the product is lost to the mother liquor, due to the good solubility of 6-APA in water.

According to US patent 3,008,956, 6-APA may be recovered from an aqueous solution by reactive extraction. In practice, this means that the aqueous solution from which the 6-APA is to be recovered is extracted with a solution of a secondary amine in an organic solvent, such as n-butanol.

The reason why the recovery process of said US patent has, after more than 30 years, still not been implemented in large-scale, commercial production processes, is that said recovery process requires the use of an excess of the secondary amine, with respect to the 6-APA to be recovered, which compound is extremely expensive. Thus, the possible gain in profits because of the increased yield of the desired product is nullified by the cost of the reagents necessary for effectuating said increased yield.

The present invention provides a process for recovering 6-aminopenicillanic acid from a mother liquor, wherein 6-aminopenicillanic acid is extracted from the mother liquor with a recovery solution, which recovery solution is collected and re-used after 6-aminopenicillanic acid is isolated therefrom.

According to the invention, it has proven to be possible to re-use a recovery solution which is used for extracting 6-APA from a mother liquor. Thus, a process has been provided wherein 6-APA can be recovered from a mother liquor in a highly efficient manner, without wasting any substantial amount of the, often expensive, recovery solution. In other words, the overall yield of 6-APA in a commercial production process for said β-lactam nucleus can be significantly increased, while at the same time the overall costs are kept low, so that the recovery of 6-APA from a mother liquor is now economically highly attractive. Also, because the recovery solution is re-used, effluent streams are greatly reduced, thus providing an additional advantage.

A suitable starting material for a process according to the invention is any aqueous solution of 6-APA. For practical reasons, a suitable starting material will generally have a concentration of 6-APA of no more than 10 grams per liter. The reason for this is that 6-APA can be more easily isolated in another way, different from the presently proposed method, from an aqueous solution having a concentration higher than 10 grams per liter by crystallization. Although it is thus not necessary to apply the present process to aqueous solutions having a 6-APA concentration of 10 grams per liter or more, it is of course very well possible.

In a preferred embodiment, a process according to the invention is part of a production process of 6-APA, wherein said compound is enzymatically prepared from penicillin G. In such a process, an aqueous solution of 6-APA is obtained, from which solution 6-APA is isolated by crystallization. The mother liquor which remains after filtration of the 6-APA crystals can suitably be used as a starting material of a process according to the invention.

As a recovery solution for extracting a mother liquor in a process according to the invention, it is preferred to use a solution of an amine or a mixture of amines. Preferred are secondary amines, in particular those that are liquid at room temperature. Particularly preferred are those secondary amines which have the formula (I): 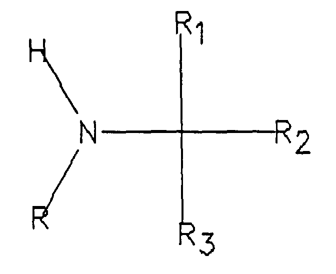 wherein R is an aliphatic group having from 8 to 16 carbon atoms and R₁, R₂, and R₃ are each alkyl groups having a total (R₁ + R₂ + R₃) of from 8 to 14 carbon atoms.

Within the class described above, a preferred group secondary amines comprises secondary amines which have a molecular weight between 325 and 395. Advantageously, a mixture of these secondary amines is used, for instance the mixture LA2 which can be purchased from Rohm and Haas. The LA2 mixture is a mixture of amines according to formula (I) having an average molecular weight of about 363, wherein R₁ and R₃ both are methyl groups, R₂ is an alkyl straight chain of 7 to 10 carbon atoms, and R is an aliphatic chain of 8 to 16 carbon atoms, the principal R chain having 12 carbon atoms.

These amines and mixtures thereof may be used in their free-base form or in the form of salts with ions such as chloride, sulfate, acetate, citrate, and so forth. The amount of such amines present in the recovery solution is from about 0.5 wt.%, preferably about 1.5 wt.%, to about 15 wt.%, preferably about 5 wt.%, based on the amount of the recovery solution. While greater amounts of the amines may be used, it has been found that this is not significantly more advantageous.

It has been found that the solvent for the recovery solution can be any solvent chosen from the group consisting of substantially water-immiscible organic solvents. Preferably, the organic solvent is chosen from the group of alcohols having the formula R₄OH, wherein R₄ is chosen from the group of alkyl and cycloalkyl groups having from 4 to 6 carbon atoms. It has been found, that the best results are obtained when one or a mixture of n-butyl, isobutyl, n-amyl and cyclohexyl alcohols is used as the organic solvent.

The amount of the solution of the amine (the recovery solution) used for extracting a mother liquor will usually be approximately equal to the amount of mother liquor that is extracted, i.e. from 0.5 to 2 volume parts of the solution of the amine to 1 volume of mother liquor. The extraction of a mother liquor with a recovery solution as described above, will usually be performed at a pH of about 6 to about 8.

After carrying out the above extraction procedure, an organic phase comprising 6-APA, and the amine, or mixture of amines, and an aqueous phase comprising various impurities are obtained. From the organic phase, the recovered 6-APA can be isolated. The aqueous phase may be discarded as a waste stream.

However, often small amounts of the organic solvent, in which little amounts of the amine and/or 6-APA may be present, will be present in the aqueous phase. It is preferred that these remains of the organic solvent are recovered. This can be done by heating, upon which the organic solvent will separate from the aqueous phase. Alternatively, the aqueous phase will be heated to an extent that the water will evaporate from the organic solvent. Advantageously, the thus recovered organic solvent may be re-used. An additional advantage is that any 6-APA, that may be present in the aqueous phase due to irregularities in the extraction of the mother liquor, can yet be recovered in this manner, together with the remains of the organic solvent.

It is preferred to isolate 6-APA from said organic phase by extraction with an aqueous solution of a mineral acid having a pH of about 0.5, preferably about 1, to about 3, preferably about 2, and subsequent crystallization. In accordance with this embodiment, the invention relates to a process for recovering 6-APA from a mother liquor comprising the steps of:
A) extracting the mother liquor with a recovery solution as described above, which results in an aqueous phase and an organic phase;
B) extracting the organic phase obtained in step A) with an aqueous solution of a mineral acid having a pH of about 1 to about 3, which results in a second aqueous phase and a second organic phase;
C) crystallizing recovered 6-APA from the second aqueous phase; and
D) collecting the second organic phase to be re-used.

Although any mineral acid has been found to be suitable, sulfuric acid is generally used for practical reasons. These practical reasons comprise the fact that aqueous solutions obtained from a production process of 6-APA from penicillin G, which is obtained from a fermentation process, usually contain phenylacetic acid, which has to be removed in order to obtain sufficiently pure 6-APA. As the usual steps of removing phenylacetic acid from the aqueous 6-APA solution involve the use of sulfuric acid, the starting material of a process according to the invention will often already comprise sulfate ions. Hence, the use of sulfuric acid as the mineral acid in the extraction of the recovery solution does not add anions of a kind, which was not already present in the starting material.

The extraction procedure of the recovery solution with an aqueous solution of a mineral acid results in a second organic phase and a second aqueous phase. The second aqueous phase comprises the desired product, 6-APA, which can be isolated by crystallization. Apart from 6-APA, this second aqueous phase will usually contain slight amounts of the organic solvent, in which little amounts of the amine may be present. Preferably, these slight amounts of the organic solvent are isolated from the second aqueous phase. A suitable way of doing so comprises neutralization of the second aqueous phase, followed by heating and separation of the organic solvent. Isolation of the organic solvent from the second organic phase assures that the end product, recovered 6-APA, will contain no significant or harmful amount of the amine.

When the mother liquor, from which 6-APA is to be recovered in a process according to the invention, is obtained from a production process of 6-APA from penicillin G as described hereinabove, it is advantageous, to carry out the crystallization, which yields the recovered 6-APA, simultaneously, preferably in the same crystallizing unit, with the crystallization of 6-APA from the aqueous solution obtained from said production process of 6-APA. This is advantageous in that a higher yield of 6-APA may be recovered from a mother liquor and in that the amount of equipment necessary for carrying out a production process of 6-APA is kept to a minimum.

The above second organic phase, resulting from the extraction procedure of the recovery solution with an aqueous solution of a mineral acid, comprises the amine, or mixture of amines, and the organic solvent. According to the invention, this second organic phase is collected and re-used, e.g. as a recovery solution. Of course, said second organic phase can advantageously be re-used as the recovery solution in a process according to the invention. However, it is also possible to re-use it as a recovery solution in a different process. Surprisingly, it has been found that a recovery solution can be re-used many times before it deteriorates unacceptably in quality. The use of a recovery solution of a deteriorated quality would result in a recovered product of unacceptable quality. According to the invention, it is therefore possible to avoid an economically undesirable large consumption of recovery solution, while at the same time a good quality of recovered product can be guaranteed.

In accordance with a highly preferred embodiment of the invention, it has been found that the deterioration of a recovery solution can be slowed down. As a result, a recovery solution can be re-used even more often. This result can be obtained when a chelating agent is present. Preferred chelating agents are chosen from the group of ethylenediaminetetraacetic acid (EDTA), ethylene glycol bis(β-aminoethyl ether) (EGTA), N,N-bis[carboxymethyl]-glycine, 1,3-diaminopropane tetraacetic acid, diethanolglycine, diethylenetriaminepentaacetic acid, diethylenetriaminepenta(methylene phosphonic acid), N,N-dihydroxyethylglycine, ethylaminotetraacetic acid, ethylenediamine-N,N'-bis(2-hydroxyphenylacetic acid), nitriloacetic acid, and salts thereof. Particularly good results have been obtained with EDTA and salts thereof.

A chelating agent may suitably be added to a solution at any stage during a process according to the invention. It may for instance be added to the mother liquor before it is extracted. It is also possible to add a chelating agent after said extraction, for instance at the time of the crystallization of the 6-APA. It has been found that the chelating agent has a beneficial influence on the quality of the recovery solution, which is to be re-used. In addition, it has been found that the 6-APA, which is recovered from a mother liquor in a process according to this embodiment of the invention is of extremely good quality, even when a recovery solution is utilized, which has been recycled for many times already.

An additional advantage of a process according to the invention is that it may be performed at low temperatures, which prevents substantial amounts of products to decompose. Preferably, a process according to the invention is performed at a temperature of from about 0°C, preferably about 5°C, to about 50°C, preferably about 35°C.

It has been found that the amount of 6-APA that is recovered from a mother liquor in a process according to the invention can be optimized by carrying out the extraction of a mother liquor with a recovery solution in countercurrent. Of course, it may also be advantageous to carry out any possible subsequent extraction steps, such as an extraction of the recovery solution with an aqueous solution of a mineral acid in countercurrent.

The amount of recovered 6-APA can be even further optimized by repeating the extractions, that are involved in the process, 2-5 times.

According to yet another preferred embodiment of the invention, the extractions involved are carried out in a centrifugal extractor. Surprisingly, it has been found that by using such an appliance, the extractions can be performed in a shorter time, which reduces the possible amount of 6-APA that is decomposed during the process. Also, the amount of recovery solution that is required, i.e. the amount of recovery solution that is in circulation during the process, is small when a centrifugal extractor is used.

The invention also concerns 6-aminopenicillanic acid obtainable by a process as outlined hereinabove.

The invention will now be elucidated by the following non-restrictive examples.

### EXAMPLES

### Method for determination of color of 6-APA in a bicarbonate solution

1.0 g 6-APA is suspended 30 ml of a 2% (by weight) solution of NaHCO₃ in water. The mixture is stirred until 6-APA is dissolved, and filtered over a Whatman GF/A glass microfiber filter. The extinction of the filtrate is measured in a spectrophotometer in a 1 cm quartz cuvet (reference is a 2% NaHCO₃ solution in water) at 425 nm.

### EXAMPLE 1

### Preparation of a solution of 6-APA

Pen G is subjected to enzymatic deacylation by adding immobilized Pen G acylase to an aqueous solution of Pen G. The pH is maintained at 8.0 by adding a solution of KOH. The concentration of Pen G is chosen so that the resulting 6-APA concentration is approximately 40 g/l. Once Pen G is converted to 6-APA, the immobilized Pen G acylase is filtered off. Next, the aqueous 6-APA solution is extracted twice with n-butanol or isobutanol at pH 1.5 to remove the phenylacetic acid.

### EXAMPLE 2

### Crystallization and isolation of 6-APA from an acidic 6-APA solution

The following equipment is used to crystallize and isolate 6-APA:
* a cylindrical flask equipped with a stirrer, cooling jacket, side outlet at 1000 ml volume, pH electrode and a dosing unit for addition of 25% ammonia
* a round bottom flask with a stirrer, cooling jacket and a side outlet at 5 l volume
* two glass filters (diameter 19 cm)
* two suction flasks (volume 10 l)
* a ventilation stove
6-APA is crystallized and isolated as follows:
- acidic water layer containing 6-APA, pH=1.5, temperature 2-5 ° C, obtained as described in example 1, is transferred to the cylindrical flask continuously
- the flow rate of the acidic 6-APA solution to the crystallizer is 6.8 l/h
- the pH in the cylindrical flask is kept at pH=4.0 by addition of 25% ammonia, and the temperature is kept at 10 °C
- the contents of the cylindrical flask are transferred to the round bottom flask by gravity. The temperature is kept at 3-5 °C
- the contents of the round bottom flask are transferred to one of the glass filters
- the mother liquor is removed by filtration until 10 l mother liquor is collected in the suction flask. At this point, the crystal slurry from the round bottom flask is transferred to the other glass filter and suction flask, and the wet cake on the filter is washed with 900 ml water.
- the washed product is dried in a ventilation stove at 35 °C. The contents of each filter are dried separately, and a number of batches is collected
- the mother liquor and washing liquid are discarded
The mother liquor is analyzed by HPLC, and contains about 3 g 6-APA/l. The flow of the mother liquor is 7.2 l/h. Therefore the loss in the mother liquor is about 100*3*7.2/(6.8*40)%= 8%.
The color of the 6-APA in a bicarbonate solution is determined as described above. Various batches are analyzed. The extinction varies from 0.003 to 0.004.

### EXAMPLE 3

### Simultaneous crystallization of 6-APA and recovery of 6-APA from mother liquor, followed by isolation

Simultaneous crystallization of 6-APA and recovery of 6-APA is executed as follows:
+ 6-APA in acidic solution obtained as described in example 1 is crystallized as described in example 2
+ mother liquor obtained as described in example 1 is extracted with n-butanol/LA2 (60 g/l LA2) at pH=7 as described below (extraction). This extraction furnishes a n-butanol/LA2 layer containing 6-APA, and a water layer containing inorganic salts and remnants 6-APA
+ The n-butanol/LA2 layer containing 6-APA is back extracted at pH=1.5 with water as described below (back extraction). This back extraction furnishes a n-butanol/LA2 layer (which is fed as such to the extraction) and an acidic water layer containing 6-APA
+ The acidic water layer is transferred to the same crystallizer as the acidic solution obtained as described in example 1.

The various process steps are described in detail:

### Crystallization:

The equipment used for crystallization is described in example 2.
6-APA is crystallized and isolated as follows:
- acidic water layer containing 6-APA, pH=1.5, temperature 2-5 ° C, obtained as described in example 1, is transferred to the cylindrical flask continuously. Flow rate: 6.8 l/h.
- concurrent with this 6-APA solution, acidic 6-APA solution from S1B (see below, back extraction) is transferred to the cylindrical flask by gravity. Flow rate: 0.8 l/h
- the pH in the cylindrical flask is kept at pH=4.0 by addition of 25% ammonia, and the temperature is kept at 10 °C
- the contents of the cylindrical flask are transferred to the round bottom flask by gravity. The temperature is kept at 3-5 °C
- the contents of the round bottom flask are transferred to one of the glass filters
- the mother liquor is removed by filtration until 10 l mother liquor is collected in the suction flask. At this point, the crystal slurry from the round bottom flask is transferred to the other glass filter and suction flask, and the wet cake on the filter is washed with 900 ml water.
- the washed product is dried in a ventilation stove at 35 °C. The contents of each filter are dried separately, and a number of batches is collected
- the mother liquor and washing liquid are transferred to the mother liquor buffer (see below, extraction)

### Extraction:

The following equipment is used for extraction of mother liquor:
* a mother liquor buffer (a 50 l drum, cooled in ice)
* 4 mixers, numbered M1E, M2E, M3E and M4E. In this case, a mixer is a beaker with a side outlet at 600 ml of the volume and equipped with a stirrer. The side outlet is connected to a settler. Mixer M1E is equipped with a pH electrode and a dosing unit for addition of 25% ammonia.
* 4 settlers, numbered S1E, S2E, S3E and S4E. In this case, a settler is a horizontal cylindrical vessel (700 ml). Each settler is equipped with an inlet (connected to the side outlet of a mixer), and two outlets. One outlet releases the upper layer, the other outlet releases the bottom layer.
* a buffer for n-butanol/LA2 (a 1000 ml beaker). The total amount of n-butanol/LA2 in extraction and back extraction is 4.5 l.
The mixers and settlers are placed above each other in alternate fashion, that is M1E is on top, above S1E, which is above M2E, which is above S2E and so on
The extraction is executed as follows:
- mother liquor from the mother liquor buffer is introduced in M4E by aid of a peristaltic pump. Flow rate: 8.5 l/h.
- upper layer from S4B (see below in description of back extraction) is introduced in M1E. Flow rate: 8.5 l/h.
- the pH in M1E is maintained at pH =7.0 by addition of 25% ammonia. Consumption 80 ml/h.
- the contents of M1E are transferred to S1E by gravity
- the upper layer from S1E is transferred to M2E by gravity
- the bottom layer from S1E is analyzed by HPLC (see below) and subsequently discarded
- the contents of M2E are transferred to S2E by gravity
- the upper layer from S2E is transferred to M3E by gravity
- the bottom layer from S2E is transferred to M1E by aid of a peristaltic pump.
- the contents of M3E are transferred to S3E by gravity
- the upper layer from S3E is transferred to M4E by gravity
- the bottom layer from S3E is transferred to M2E by aid of a peristaltic pump.
- the contents of M4E are transferred to S4E by gravity
- the upper layer from S4E is transferred to the n-butanol/LA2 buffer by gravity
- the bottom layer from S4E is transferred to M3E by aid of a peristaltic pump.
- the level in the n-butanol/LA2 buffer is kept to about 400 ml by addition of n-butanol
- the temperature is 10-15 °C during the extraction procedure

### Back extraction:

The following equipment is used for back extraction of the n-butanol/LA2 containing 6-APA:
* 4 mixers, numbered M1B, M2B, M3B and M4B. In this case, a mixer is a round bottom flask with a side outlet at 250 ml of the volume, equipped with a stirrer and a cooling jacket. The side outlet is connected to a settler. Mixer M1B is equipped with a pH electrode and a dosing unit for addition of 3 M sulfuric acid.
* 4 settlers, numbered S1B, S2B, S3B and S4B. In this case, a settler is a horizontal cylindrical vessel (350 ml). Each settler is equipped with an inlet (connected to the side outlet of a mixer), and two outlets. One outlet releases the upper layer, the other outlet releases the bottom layer. The mixers and settlers are placed above each other in alternate fashion, that is M1B is on top, above S1B, which is above M2B, which is above S2B and so on.
The back extraction is executed as follows:
- water is introduced in M4B. Flow rate: 0.8 l/h
- n-butanol/LA2 from the buffer (see description of extraction) is introduced in M1B. Flow rate: 8.5 l/h.
- the temperature in the mixers is kept at 2-5 °C
- the pH in M1B is maintained at pH =1.5 by addition of 3 M sulfuric acid. Consumption: 0.2 l/h.
- the contents of M1B are transferred to S1B by gravity
- the upper layer from S1B is transferred to M2B by gravity
- the bottom layer from S1B is transferred to the cylindrical flask in the crystallization procedure (see above) by gravity
- the contents of M2B are transferred to S2B by gravity
- the upper layer from S2B is transferred to M3B by gravity
- the bottom layer from S2B is transferred to M1B by aid of a peristaltic pump.
- the contents of M3B are transferred to S3B by gravity
- the upper layer from S3B is transferred to M4B by gravity
- the bottom layer from S3B is transferred to M2B by aid of a peristaltic pump.
- the contents of M4B are transferred to S4B by gravity
- the upper layer from S4B is transferred to M1E (see above, extraction) by the aid of a peristaltic pump
- the bottom layer from S4B is transferred to M3B by aid of a peristaltic pump.

### Results:

# The water layer from S1E is analyzed by HPLC. The 6-APA concentration is about 0.8 g/l. The flow of this stream is 9 l/h. The loss is therefore 100*9*0.8/(6.8*40)=2.6%.
# The color of the 6-APA in a bicarbonate solution is determined as described above. Various batches are analyzed. In the following table the extinctions as a function of run time of the experimental set up are presented (run time = 0 is the start of introduction of mother liquor in the extraction):

| run time (h) | extinction |
|---|---|
| 0 | 0.004 |
| 1 | 0.006 |
| 2.5 | 0.006 |
| 4 | 0.009 |
| 5 | 0.010 |
| 8 | 0.010 |
| 11 | 0.010 |
| 13 | 0.010 |
| 17 | 0.010 |
| 20 | 0.012 |
| 24 | 0.010 |
| 26 | 0.011 |
| 33 | 0.011 |
| 50 | 0.010 |

### EXAMPLE 4

### Simultaneous crystallization of 6-APA and recovery of 6-APA from mother liquor, followed by isolation

Simultaneous crystallization of 6-APA and recovery of 6-APA is executed as follows:
+ 6-APA in acidic solution obtained as described in example 1 is crystallized as described in example 2
+ mother liquor obtained as described in example 1 is extracted with n-butanol/LA2 (60 g/l LA2) at pH=7 as described below (extraction). This extraction furnishes a n-butanol/LA2 layer containing 6-APA, and a water layer containing inorganic salts and remnants 6-APA
+ The n-butanol/LA2 layer containing 6-APA is back extracted at pH=1.5 with water as described below (back extraction). This back extraction furnishes a n-butanol/LA2 layer (which is fed as such to the extraction) and an acidic water layer containing 6-APA
+ The acidic water layer is transferred to the same crystallizer as the acidic solution obtained as described in example 1
+ After continuous operation for 5 hours, Na₂EDTA.2H₂O is added to the 25% ammonia used in crystallization of the acidic 6-APR solutions. 264 mg Na₂EDTA.2H₂O per liter 25% ammonia is added, and this EDTA in ammonia solution is used for pH control in the crystallization of 6-APA.

The various process steps are described in detail:

### Crystallization:

The equipment used for crystallization is described in example 2.
6-APA is crystallized and isolated as follows:
- acidic water layer containing 6-APA, pH=1.5, temperature 2-5 ° C, obtained as described in example 1, is transferred to the cylindrical flask continuously. Flow rate: 6.8 l/h.
- concurrent with this 6-APA solution, acidic 6-APA solution from S1B (see below, back extraction) is transferred to the cylindrical flask by gravity. Flow rate: 0.8 l/h.
- the pH in the cylindrical flask is kept at pH=4.0 by addition of 25% ammonia, and the temperature is kept at 10 °C
- the contents of the cylindrical flask are transferred to the round bottom flask by gravity. The temperature is kept at 3-5 °C
- the contents of the round bottom flask are transferred to one of the glass filters
- the mother liquor is removed by filtration until 10 l mother liquor is collected in the suction flask. At this point, the crystal slurry from the round bottom flask is transferred to the other glass filter and suction flask, and the wet cake on the filter is washed with 900 ml water.
- the washed product is dried in a ventilation stove at 35 °C. The contents of each filter are dried separately, and a number of batches is collected
- the mother liquor and washing liquid are transferred to the mother liquor buffer (see below, extraction)

### Extraction:

The following equipment is used for extraction of mother liquor:
* a mother liquor buffer (a 50 l drum, cooled in ice)
* 2 mixers, numbered M1E and M2E. In this case, a mixer is a beaker with a side outlet at 600 ml of the volume and equipped with a stirrer. The side outlet is connected to a settler. Mixer M1E is equipped with a pH electrode and a dosing unit for addition of 25% ammonia.
* 2 settlers, numbered S1E and S2E. In this case, a settler is a horizontal cylindrical vessel (700 ml). Each settler is equipped with an inlet (connected to the side outlet of a mixer), and two outlets. One outlet releases the upper layer, the other outlet releases the bottom layer.
* a buffer for n-butanol/LA2 (a 1000 ml beaker). The total amount of n-butanol/LA2 in extraction and back extraction is 2.5 l.
The mixers and settlers are placed above each other in alternate fashion, that is M1E is on top, above S1E, which is above M2E, which is above S2E.
The extraction is executed as follows:
- mother liquor from the mother liquor buffer is introduced in M2E by aid of a peristaltic pump. Flow rate: 8.5 l/h.
- upper layer from S1B (see below in description of back extraction) is introduced in M1E. Flow rate: 8.5 l/h..
- the pH in M1E is maintained at pH=7.0 by addition of 25% ammonia. Consumption 80 ml/h.
- the contents of M1E are transferred to S1E. by gravity
- the upper layer from S1E is transferred to M2E by gravity
- the bottom layer from S1E is analyzed by HPLC (see below) and subsequently discarded
- the contents of M2E are transferred to S2E by gravity
- the upper layer from S2E is transferred to the n-butanol/LA2 buffer by gravity
- the bottom layer from S2E is transferred to M1E by aid of a peristaltic pump.
- the level in the n-butanol/LA2 buffer is kept to about 400 ml by addition of n-butanol
- the temperature is 10-15 °C during the extraction procedure

### Back extraction:

The following equipment is used for back extraction of the 6-APA containing n-butanol/LA2:
* 1 mixer, numbered M1B. In this case, a mixer is a round bottom flask with a side outlet at 250 ml of the volume, equipped with a stirrer and a cooling jacket. The side outlet is connected to a settler. Mixer M1B is equipped with a pH electrode and a dosing unit for addition of 3 M sulfuric acid.
* 1 settler, numbered S1B. In this case, a settler is a horizontal cylindrical vessel ( 350 ml). The settler is equipped with an inlet (connected to the side outlet of the mixer), and two outlets. One outlet releases the upper layer, the other outlet releases the bottom layer.
The mixer and settler are placed above each other, M1B is above S1B.
The back extraction is executed as follows:
- water is introduced in M1B. Flow rate: 0.8 l/h
- n-butanol/LA2 from the buffer (see description of extraction) is introduced in M1B. Flow rate: 8.5 l/h.
- the temperature in the mixer is kept at 2-5 °C
- the pH in M1B is maintained at pH =1.5 by addition of 3 M sulfuric acid. Consumption: 0.2 l/h.
- the contents of M1B are transferred to S1B by gravity
- the upper layer from S1B is transferred to M1E (see above, extraction) by aid of a peristaltic pump
- the bottom layer from S1B is transferred to the crystallizer (see above) by gravity.

### Results:

# The water layer from S1E is analyzed by HPLC. The 6-APA concentration is about 1.8 g/l. The flow of this stream is 9 l/h. The loss is therefore 100*9*1.8/(6.8*40)=6%.
# The color of the 6-APA in a bicarbonate solution is determined as described above. Various batches are analyzed. In the following table the extinctions as a function of run time of the experimental set up are presented (run time = 0 is the start of introduction of mother liquor in the extraction)

| run time (h) | extinction | |
|---|---|---|
| 0 | 0.004 | |
| 1 | 0.004 | |
| 2 | 0.005 | |
| 3 | 0.006 | |
| 4 | 0.006 | |
| 5 | 0.006 | From this moment on, 264 mg Na₂EDTA.2H₂O per liter is dissolved in the 25% ammonia used for pH control in crystallization |
| 6 | 0.005 | |
| 7 | 0.005 | |
| 8 | 0.003 | |
| 9 | 0.003 | |
| 10 | 0.003 | |
| 11 | 0.003 | |

# The color of the n-butanol/LA2 intensifies from yellow to brown before addition of EDTA to the ammonia solution, and gradually changes from brown to yellow after addition of EDTA to the ammonia solution. The color of the mother liquor changes from slightly yellow to gray/blue before addition of EDTA to the ammonia solution, changes back to slightly yellow after addition of EDTA to the ammonia solution.

### EXAMPLE 5

### Simultaneous crystallization of 6-APA in the presence of EDTA and recovery of 6-APA from mother liquor, followed by isolation

Simultaneous crystallization of 6-APA and recovery of 6-APA is executed as follows:
+ 6-APA in acidic solution obtained as described in example 1 is crystallized as described in example 2. pH control in the crystallizer is not as in example 2 done with 25% ammonia, but with 25% ammonia containing 264 mg Na₂EDTA.2H₂O per liter.
+ mother liquor obtained as described in example 1 is extracted with n-butanol/LA2 (60 g/l LA2) at pH=7 as described below (extraction). This extraction furnishes a n-butanol/LA2 layer containing 6-APA, and a water layer containing inorganic salts and remnants 6-APA
+ the water layer coming from the extraction is warmed and n-butanol is separated from the mixture. The n-butanol is added to the n-butanol/LA2, the water layer is analyzed and discarded.
+ The n-butanol/LA2 layer containing 6-APA from the extraction is back extracted at pH=1.5 with water as described below (Back extraction). This back extraction furnishes a n-butanol/LA2 layer (which is fed as such to the extraction) and an acidic water layer containing 6-APA
+ The acidic water layer is neutralized to pH=7 with ammonia, and warmed. n-butanol is separated and added to the n-butanol/LA2. The neutralized water layer after separation of n-butanol is transferred to the same crystallizer as the acidic solution obtained as described in example 1.

The various process steps are described in detail:

### Crystallization:

The equipment used for crystallization is described in example 2.
6-APA is crystallized and isolated as follows:
- acidic water layer containing 6-APA, pH=1.5, temperature 2-5 ° C, obtained as described in example 1, is transferred to the cylindrical flask continuously. Flow rate: 6.8 l/h.
- concurrent with this 6-APA solution, neutralized 6-APA solution from the neutralization step (see below) is transferred to the cylindrical flask by gravity. Flow rate: 0.9 l/h.
- the pH in the cylindrical flask is kept at pH=4.0 by addition 25% ammonia, containing 264 mg Na₂EDTA.2H₂O per liter. The temperature is kept at 10 °C
- the contents of the cylindrical flask are transferred to the round bottom flask by gravity. The temperature is kept at 3-5 °C
- the contents of the round bottom flask are transferred to one of the glass filters
- the mother liquor is removed by filtration until 10 l mother liquor is collected in the suction flask. At this point, the crystal slurry from the round bottom flask is transferred to the other glass filter and suction flask, and the wet cake on the filter is washed with 900 ml water.
- the washed product is dried in a ventilation stove at 35 °C. The contents of each filter are dried separately, and a number of batches is collected
- the mother liquor and washing liquid are transferred to the mother liquor buffer (see below, extraction)

### Extraction:

The following equipment is used for extraction of mother liquor:
* a mother liquor buffer (a 50 l drum, cooled in ice)
* 4 mixers, numbered M1E, M2E, M3E and M4E. In this case, a mixer is a beaker with a side outlet at 600 ml of the volume and equipped with a stirrer. The side outlet is connected to a settler. Mixer M1E is equipped with a pH electrode and a dosing unit for addition of 25% ammonia.
* 4 settlers, numbered S1E, S2E, S3E and S4E. In this case, a settler is a horizontal cylindrical vessel (700 ml). Each settler is equipped with an inlet (connected to the side outlet of a mixer), and two outlets. One outlet releases the upper layer, the other outlet releases the bottom layer.
* a buffer for n-butanol/LA2 (a 1000 ml beaker). The total amount of n-butanol/LA2 in extraction and back extraction is 4.5 l.
The mixers and settlers are placed above each other in alternate fashion, that is M1E is on top, above S1E, which is above M2E, which is above S2E and so on.
The extraction is executed as follows:
- mother liquor from the mother liquor buffer is introduced in M4E by aid of a peristaltic pump. Flow rate: 8.5 l/h.
- upper layer from S4B (see below in description of back extraction) is introduced in M1E. Flow rate: 8.5 l/h.
- the pH in M1E is maintained at pH =7 by addition of 25% ammonia. Consumption 80 ml/h.
- the contents of M1E are transferred to S1E by gravity
- the upper layer from S1E is transferred to M2E by gravity
- the bottom layer from S1E is transferred to a round bottom flask (see below, coalescence of the water layer from the extraction)
- the contents of M2E are transferred to S2E by gravity
- the upper layer from S2E is transferred to M3E by gravity
- the bottom layer from S2E is transferred to M1E by aid of a peristaltic pump.
- the contents of M3E are transferred to S3E by gravity
- the upper layer from S3E is transferred to M4E by gravity
- the bottom layer from S3E is transferred to M2E by aid of a peristaltic pump.
- the contents of M4E are transferred to S4E by gravity
- the upper layer from S4E is transferred to the n-butanol/LA2 buffer by gravity
- the bottom layer from S4E is transferred to M3E by aid of a peristaltic pump.
- the level in the n-butanol/LA2 buffer is kept to about 400 ml by addition of n-butanol
- the temperature is 10-15 °C during the extraction procedure

Coalescence of the water layer from the extraction:
In order to remove dissolved n-butanol from the water layer from S1E from the extraction, the water layer is warmed up. n-butanol separates, and is transferred back to the n-butanol/LA2 buffer. The following equipment is used.
* a round bottom flask equipped with a stirrer, a heating jacket and a side outlet at 250 ml volume
* a settler. In this case, a settler is a horizontal cylindrical vessel (350 ml). The settler is equipped with an inlet (connected to the side outlet of the round bottom flask), and two outlets. One outlet releases the upper layer, the other outlet releases the bottom layer.
The coalescence of the water layer is executed as follows:
- the bottom layer from S1E is transferred to the round bottomed flask by gravity and warmed to 30 °C. Due to the rise in temperature, dissolved n-butanol separates from the mixture.
- the contents of the round bottom flask are transferred to the settler by gravity
- the upper layer from the settler is transferred to the n-butanol/LA2 buffer (see above, extraction) by gravity
- the bottom layer is analyzed by HPLC (see below) and discarded

### Back extraction:

The following equipment is used for back extraction of the 6-APA containing n-butanol/LA2:
* 4 mixers, numbered M1B, M2B, M3B and M4B. In this case, a mixer is a round bottom flask with a side outlet at 250 ml of the volume, equipped with a stirrer and a cooling jacket. The side outlet is connected to a settler. Mixer M1B is equipped with a pH electrode and a dosing unit for addition of 3 M sulfuric acid.
* 4 settlers, numbered S1B, S2B, S3B, S4B. In this case, a settler is a horizontal cylindrical vessel ( 350 ml). Each settler is equipped with an inlet (connected to the side outlet of a mixer), and two outlets. One outlet releases the upper layer, the other outlet releases the bottom layer. The mixers and settlers are placed above each other in alternate fashion, that is M1B is on top, above S1B, which is above M2B, which is above S2B and so on.
The back extraction is executed as follows:
- water is introduced in M4B. Flow rate: 0.8 l/h
- n-butanol/LA2 from the buffer (see description of extraction) is introduced in M1B. Flow rate: 8.5 l/h.
- the temperature in the mixers is kept at 2-5 °C
- the pH in M1B is maintained at pH =1.5 by addition of 3 M sulfuric acid. Consumption: 0.2 l/h.
- the contents of M1B are transferred to S1B by gravity
- the upper layer from S1B is transferred to M2B by gravity
- the bottom layer from S1B is transferred to the round bottom flask in the neutralization of the water from the back extraction (see below) by gravity
- the contents of M2B are transferred to S2B by gravity
- the upper layer from S2B is transferred to M3B by gravity
- the bottom layer from S2B is transferred to M1B by aid of a peristaltic pump.
- the contents of M3B are transferred to S3B by gravity
- the upper layer from S3B is transferred to M4B by gravity
- the bottom layer from S3B is transferred to M2B by aid of a peristaltic pump.
- the contents of M4B are transferred to S4B by gravity
- the upper layer from S4B is transferred to M1E (see above) by the aid of a peristaltic pump
- the bottom layer from S4B is transferred to M3B by aid of a peristaltic pump.

Neutralization of the water layer from the back extraction: In order to remove n-butanol from the water layer from S1B from the back extraction, the water layer is neutralized, and warmed. n-butanol is separated, and returned to the n-butanol/LA2 buffer (see above, extraction).
The following equipment is used:
* a round bottom flask equipped with a stirrer, a heating jacket ,a side outlet at 250 ml volume, a pH electrode and a dosing unit for addition of 25% ammonia
* a settler. In this case, a settler is a horizontal cylindrical vessel (350 ml). The settler is equipped with an inlet (connected to the side outlet of the round bottom flask), and two outlets. One outlet releases the upper layer, the other outlet releases the bottom layer.
* a neutralized product layer buffer (a cylindrical vessel with a jacket, a stirrer and a side outlet at 1 l volume)
Neutralization and separation is executed as follows:
- the bottom layer from S1B is transferred to the round bottomed flask. The mixture is warmed to 20 °C and the pH is maintained at pH=7.0. Due to the rise in temperature, dissolved n-butanol separates from the mixture.
- the contents of the round bottom flask are transferred to the settler by gravity
- the upper layer from the settler is transferred to the n-butanol/LA2 buffer (see above, extraction) by gravity.
- the bottom layer is transferred to the neutralized product layer buffer by gravity
- the contents of this buffer are transferred to the crystallizer (see below) by the aid of a peristaltic pump. Flow rate: 0.8 l/h.

### Results:

# The water layer from S1E is analyzed by HPLC. The 6-APA concentration is about 0.6 g/l. The flow of this stream is 8.6 l/h. The loss is therefore 100*0.6*8.6/(6.8*40)=2%.
# The experiment is executed for 137 hours. During this period, the color of the n-butanol/LA2 has remained yellow, and the color of the mother liquor has remained slightly yellow.
# The color of the 6-APA in a bicarbonate solution is determined as described above. Various batches are analyzed. The extinction varies between 0.004 and 0.006. No increase with increasing run time is observed.

## Claims

1. A process for recovering 6-aminopenicillanic acid from a mother liquor, wherein 6-aminopenicillanic acid is extracted from the mother liquor with a recovery solution, which recovery solution is collected and re-used after 6-aminopenicillanic acid is isolated therefrom.

2. A process according to claim 1, wherein the recovery solution comprises an amine or a mixture thereof.

3. A process according to claim 2, wherein the recovery solution comprises a secondary amine or a mixture thereof.

4. A process according to claim 3, wherein the secondary amine is liquid at room temperature.

5. A process according to claim 4, wherein the recovery solution comprises a secondary amine having the formula (I): 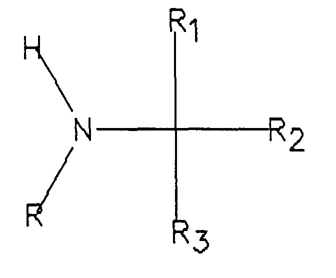 wherein R is an aliphatic group having from 8 to 16 carbon atoms and R₁, R₂, and R₃ are each alkyl groups having a total (R₁ + R₂ + R₃) of from 8 to 14 carbon atoms.

6. A process according to claim 5, wherein the recovery solution comprises a secondary amine having a molecular weight between 325 and 395.

7. A process according to any of the preceding claims, wherein the recovery solution comprises a substantially water-immiscible organic solvent.

8. A process according to claim 7, wherein the organic solvent is chosen from the group of alcohols having the formula R₄OH, wherein R₄ is chosen from the group of alkyl and cycloalkyl groups having from 4 to 6 carbon atoms.

9. A process according to claim 8, wherein the organic solvent is chosen from the group of n-butyl, isobutyl, n-amyl and cyclohexyl alcohols.

10. A process according to claims 2-9, wherein the amine is present in the recovery solution in an amount of about 0.5 wt.%, preferably about 1.5 wt.%, to about 15 wt.%, preferably about 5 wt.%, based on the amount of the recovery solution.

11. A process according to any of the preceding claims, wherein the extraction of the mother liquor with a recovery solution results in an aqueous phase and an organic phase, which aqueous phase is treated in order to isolate remains of the recovery solution therefrom.

12. A process according to any of the preceding claims, wherein 6-aminopenicillanic acid is isolated from the recovery solution by extraction with an aqueous solution of a mineral acid having a pH of about 0.5 to about 3, and subsequent crystallization.

13. A process according to claim 12, wherein the extraction of the recovery solution with an aqueous solution of a mineral acid results in an aqueous phase and an organic phase, which aqueous phase is treated in order to isolate remains of the recovery solution therefrom.

14. A process according to any of the preceding claims, wherein the temperature is from about 0°C, preferably about 5°C, to about 50°C, preferably about 35°C.

15. A process according to any of the preceding claims, wherein a chelating agent is present.

16. A process according to claim 15, wherein a compound chosen from the group of ethylenediaminetetraacetic acid (EDTA), ethylene glycol bis(β-aminoethyl ether) (EGTA), N,N-bis[carboxymethyl]glycine, 1,3-diaminopropane tetraacetic acid, diethanolglycine, diethylenetriaminepentaacetic acid, diethylenetriaminepenta(methylene phosphonic acid), N,N-dihydroxyethylglycine, ethylaminotetraacetic acid, ethylenediamine-N,N'-bis(2-hydroxyphenylacetic acid), nitriloacetic acid, and salts thereof is present.

17. A process according to any of the preceding claims, wherein the mother liquor is obtained after crystallization of 6-aminopenicillanic acid from an aqueous solution obtained in a process, wherein 6-aminopenicillanic acid is enzymatically prepared from penicillin G.

18. A process wherein the crystallization of recovered 6-aminopenicillanic acid according to claim 12 is carried out simultaneously with the crystallization of 6-aminopenicillanic acid obtained from its enzymatic preparation from penicillin G according to claim 17.

19. A process according to any of the preceding claims, wherein the extraction is carried out in countercurrent.

20. A process according to any of the preceding claims, wherein the extraction is repeated about 2-5 times.

21. A process according to any of the preceding claims, wherein the extraction is carried out in a centrifugal extractor.

22. A process according to any of the preceding claims, wherein the recovery solution is re-used in a process for recovering 6-aminopenicillanic acid from a mother liquor.

23. 6-aminopenicillanic acid obtainable by a process according to any of the preceding claims.

24. Use of a chelating agent for improving the quality of 6-aminopenicillanic acid in a process for recovering 6-aminopenicillanic acid from a mother liquor.
